# EUROPEAN PATENT APPLICATION

(11) **EP 2 805 667 A1**
(43) Date of publication of application: **26.11.2014**
(21) Application number: 12865692.3
(22) Date of filing: 18.01.2012
(51) Int. Cl.: A61B 1/04, A61B 1/06, A61B 1/303, A61B 5/00, G01N 21/64

(54) **COLPOSTEREOSCOPE FOR PHOTODYNAMIC DIAGNOSIS (PDD) OF DISEASES OF THE FEMALE GENITAL TRACT AND EARLY DETECTION OF NEOPLASTIC LESIONS**

(71) Applicant: Diaz Sánchez, Joel Gerardo, C.P. 03800 México D.F. (MX); Zertuche Zuani, Jose Gerardo, Delegacion Guauhtemoc 06700 México D.F. (MX)
(72) Inventor: Diaz Sánchez, Joel Gerardo, C.P. 03800 México D.F. (MX); Zertuche Zuani, Jose Gerardo, Delegacion Guauhtemoc 06700 México D.F. (MX)
(74) Representative: Ahmad, Sheikh Shakeel
(86) International application number: PCT/MX2012/000006
(87) International publication number: WO 2013/109130

(57) **Abstract**

The invention, namely a colpostereoscope for photodynamic diagnosis (PDD) of diseases of the female genital tract and early detection of neoplastic lesions, also functions as a standard colpostereoscope, using the current technique of colposcopy, but it also has the ability to make observations by means of the phenomenon of fluorescence owing to the fact that it has variable linearity filter systems not only in the excitation parts but also in the suppression parts and can therefore can be used universally with any fluorescent compound or medicinal product, as in photodynamic diagnosis (PDD). The display on the monitor is three-dimensional owing to the use of two video cameras with a DLP (Digital Light Processing) and HDTV (High Definition Television) systems with the use of active lenses. This equipment offers health benefits to women owing to the fact that after just one consultation it is possible to carry out a variety of actions to combat sexually transmitted diseases at low financial cost and also to provide early prevention of cervical cancer.

## Description

### Technical Field

I. Medical field: a)Medical Imaging, b) Photodynamic Diagnosis (PDD)
II. Technology field: a) Surgical microscopes, b) Stereomicroscopes, c) Medicine-applied Optics, d) Medical Instruments and Equipment

### State of the art

Since the second half of the nineteenth century, photonics and electronics technologies have led to a plethora of scientific and technological developments for medical diagnosis, resulting in new devices with which research and a better understanding about the interaction of medical instrumentation, optics and electronics with biological tissues belonging to the field of immunohistochemistry and pathology have been conducted, targeting, as in our case, the study, diagnosis and treatment of female genital tract.

As sexually transmitted diseases are currently considered emerging, there has been accordingly an increasing demand to solve women's genital tract diseases, leading to new medical device development in the last twenty years focused in medical diagnosis; however, these new methods have various disadvantages, including the high economic cost with their use, as in developing countries is almost impossible to reach the required technological level.

Examples of these high-tech diagnosis methods include: fluorescence spectroscopy and optical coherence tomography and many others.

Colposcopy has been a method which is somehow economical for sexually transmitted disease diagnosis as it is a method for direct visualization allowing a quick observation of disease signs, and especially human papillomavirus-produced neoplasms.

The instrument used to perform a gynecological examination colposcopy in the genital tract is commonly called a colposcope which increases the image size, that is, a stereoscopic microscope taking advantage of vision with both eyes facing three-dimensional images by two separate eyepieces, similar to an angle formed between the eye visual axes (between 7 to 12 degrees) providing two different images, one for each eye, and a three-dimensional perception, this being indispensable to perform several manual operations implemented in the genital tract. In the light of above, the so-called colposcope correct name is "colpostereoscope" as in present case.

The colpostereoscope was invented in Germany in 1925 by Dr. Hans Hinselmann who dissatisfied with the used scanning means of that time and intending to discover the initial forms of female genital tract diseases, combined a powerful light source with a stereoscopic zoom system for observation. The colpostereoscope has virtually remained unchanged since 1925 when it was invented.

The authors of this paper previously invented the "actinic light colposcope" with WIPO International Publication number: WO 2005/039403 Al, for early diagnosis of lesions caused by human papilloma virus, known as neoplasms which are cervical cancer precursors.

The colpostereoscopes are currently used in routine gynecological examination for purposes of diagnosis, however, any of these colpostereoscopes having simultaneous functions of diagnosis and treatment is not available. The new colposcope claimed herein as "photodynamic diagnosis colpostereoscope (PDD)" not only further improves the sensitivity and specificity in sexually transmitted disease diagnosis, as it further combines current colposcope technique diagnosis with the use of a number of fluorescent or photosensitive dyes used in photodynamic diagnosis (PDD) and in turn, through optical and electronic devices which could be useful as application for the next clinical step for an efficient treatment, allowing a successful treatment of various genital tract diseases, being also highly effective in treating precursor neoplasms applied to cervical cancer.

### Background of the Invention

For a proper understanding of the operation and benefits of this new apparatus, the concepts required thereto, including the related problems, are firstly described.

Sexually transmitted diseases (STD) are those which are transmitted during sexual intercourse being usually asymptomatic and where any sexually active person is at risk of acquiring them.

There are several types of pathogens that produce them, including: mycoses producing fungi, bacteria, protozoa and viruses.

We found the combination of multiple pathogens in most of infected cases. Vaginosis and cervicitis remain as the most common worldwide, high economic impact, female genital tract disorders in reproductive-age women.

The human papilloma virus in these diseases requires special attention because it is the cause of neoplasms, which are the cervical cancer precursor lesions.

The virus genus *Papilloma* is too long, being DNA viruses that infect many animal species, including humans. There are over 100 types of these known as Human Papilloma Virus (HVP).

Interest in these viruses has increased gradually since 1970, when they had firstly an attributed role in cervical cancer etiology.

Cervical cancer is a common type of cancer in women, being a change in the cervix epithelial tissue cells and also in vaginal walls and vulva. These cells are initially normal and they then gradually become precancerous. Tissues undergo changes before cancer cells are present in the cervix, and abnormal cells called koilocytes begin to appear, this process known as dysplasia or cervical intraepithelial neoplasia (CIN).

There are over 30 types of HVP virus having the ability to infect the lower female genital tract; benign virus called "low risk" and other oncogenic called "high risk" are within these types. The high-risk viral type in America has been the HPV 16 Asian-American variety in about 50% of cases.

Cervical cancer is one of the most common cancers in women. More than 500,000 cases per year are estimated worldwide. About 80% of these occur in developing countries. The high event incidence reflects poor programs in early disease detection, and a lack of new medical devices for diagnosis and treatment thereof.

Fluorescence is a physical phenomenon which under photon or light energy irradiation results in an irradiated compound which emits energy in a different wave length from that being exposed to, this being the principle of the Stokes-Adams optics law which, applied to an optical system with exciter and suppression filters allows locating this compound within a live tissue in high contrast overlaid on a black background. Chemical compounds serving for this purpose are called fluorescent dyes or fluorochromes such as fluorescein isothiocyanate (FITC), acridine orange, toluidine blue, Bengal Pink, Green Fluorescent Proteins and many other harmless for in vivo study of animal tissues, those which we have assayed with.

Hematoporphyrins started also their use in cancer research in 1914, these dyes and fluorochromes being called photosensitizers. Many attempts were then flawed due to impurities present in these photosensitizers.

Nowadays it is said that photodynamic diagnosis (PDD) is considered an embodiment in optical fluorescence systems based on biological material photo-oxidation induced by incubating a precursor for a photosensitizing agent, which is selectively deposited within target cells or blank cells, in order to destroy selectively damaged or altered cells in their nuclear structure, such as in neoplasms.

In 1969, Richard Lipson and Gregorie observed that hematoporphyrins derivatives (HPD) could be used as photosensitizing agents on tumor cells as these agents have a mitochondrial specificity mechanism, being deposited in neoplastic tissue which when lighted up, those are selectively fluoresced in healthy adjacent tissues and is used as a diagnosis method, currently known as photodynamic diagnosis (PDD) since by fluorescence emitted by a photosensitizer tumor tissues may be detected, being clearly identified from healthy tissues.

Most commonly used photosensitizing agents are 5-aminolevulinic acid (ALA), methyl aminolevulinate (MAL) and hexaminolevulinate (RAL). ALA is considered the first step in the metabolic process of hemo-group formation, then it may actually not be a photosensitizing agent by itself, however, its structure is crucial as a protoporphyrin IX (PpIX) precursor which is indeed a photosensitizing agent and when under normal conditions, ALA is firmly controlled by a feedback direct mechanism through Ala synthetase present in hemo-cell nuclei. When ALA is intracellularly activated PpIX production is increased, which in turn becomes into hemo through a ferrochelatase adding iron ions to PpIX. Such photosensitizing agents may be administered by enteral, parenteral and topical route.

The method we use for light energy application and transportation in photodynamic diagnosis is by Kohler illumination, directly by lenses and mirrors or instead by the use of optical fibers. An optical fiber is a tube whereby light is transmitted through its ends, a physical phenomenon known as refraction. This knowledge is not new since physicist John Tyndall in England demonstrated in nineteenth century that water from a tank comprising an internal light source, when expelled through a hole, the water jet led light to the receiving vessel.

Image transmission through tubes was made by radio experimenter Clarence Hansel and television pioneer John Logie Baird in 1920; the first uses of these tubes were for medical examination purposes; the first flexible gastroscope was released years later in 1956.

Nowadays, several optical fiber types are available in market, whether in fiber bundles or mono-cable, which are widely used in optical field for multiple purposes depending on their characteristics.

The latest optical fibers for medical applications, given their high light flux conduction, flexibility and durability, contain fluorinate, ethylene and propylene resins (FEP), which are under experimentation in this field in Australia.

### Problem

Infectious etiology female genital tract diseases including the lesions caused by human papillomavirus (HPV), which are known as cervical intraepithelial neoplasm (CIN) are often undiagnosed and not easily treated. This is due to a lack of equipment and tests in doctor's office setting for easing the diagnosis of said diseases, causing that the gynecologist turns to laboratory tests with wide margin of error, or to the use of sophisticated and high cost equipment, for example, those of spectroscopy and optical coherence tomography requiring a long training and only available in industrialized countries but not in developing countries due to a number of factors. This results in patients suffering of long waiting times and multiple doctor visits before achieving a suitable disease treatment which often is out of time for disease prevention.

### Objective of present invention.

In order to solve above problems, the creation of this new invention: "Photodynamic diagnosis (PDD) colpostereoscope for diseases of the female genital tract and neoplastic lesion early detection" was envisaged, which performs an early diagnosis and sends the patient to her clinic for timely treatment of female genital tract infectious diseases, as well as neoplasms caused by human papilloma virus that cause cervical cancer, practically at the same site and day when patient is explored.

Costly studies or long-term processes and procedures are prevented with the new colpostereoscope thus providing highly reliable results regarding detection of sexually transmitted diseases and neoplasms caused by human papilloma virus (HPV) by applying the fluorescence phenomenon.

The new colpostereoscope also works as a common colpostereoscope.

### Detailed description of the apparatus

The invention described and detailed in this document basically forms a colpostereoscope consisting of:
A head lighting system having a variable linear type excitation filter system is present in the head lighting system, capable of choosing any light frequency range to generate a light beam to any fluorescent compound located at the female genital tract, these compounds may be: fluochromes, "in vivo" use fluorescent proteins of any color, or chemical dyes for fluorescence. This filter covers a range from 400 nanometers to 1080 nanometers, the light beam band capable of being selected every 20 nanometers.

In order to analyze the frequency emitted by fluorescent compounds and to eliminate wavelengths produced by the excitation light, two suppressor or barrier filters have been assembled on colpostereoscope head, one for each observation optical axis. These filters are also of variable linearity for accurately adjusting the emission beams issued from the compound to be reacted in the dyed female genital tract. These filters span a light spectrum from 400 to 700 nanometers, which is the visible light spectrum, and frequencies may be staggered every 20 nanometers.

Two diaphragms are also located on the head in order to match what is being observed and also for watching the depth of field, one diaphragm at each optical axis with manual aperture controller.

The three-dimensional observation in this novel colpostereoscope may be by direct observation through the eyepieces as usual in these devices, or by the novel way of having two high-resolution video cameras located on the head, each camera being located at each visual axis. The three-dimensional vision video system is: DLP (digital light processing) HDTV (high definition television) with the use of three-dimensional active lenses.

The head-located lighting system described above in this photodynamic diagnosis colpostereoscope, also has another light box for optical fiber use.

A light source box with focusable optical system is targeted to a medical grade optical fiber or to the novel low cost and high quality optical fiber made of ethylene and propylene fluorinate resin (FEP). As with the head in this optical system, a variable linearity filter is also placed within the optical system being capable of choosing any light frequency range for sending a light beam through the optical fiber to any compound or drug within the female genital tract for photodynamic diagnosis application, this filter spans a range from 400 nanometers to 700 nanometers, with a light beam band which may be chosen every 20 nanometers thus providing a virtually universal equipment application to any drug or dye intended for diagnosis by a fluorescence phenomenon.

It is important to show and to emphasize that the scope of this invention goes beyond current knowledge and diagnoses in the field of Colposcopy, and this equipment is a strong tool for future innovations in diagnosis, opening a new gate in female genital tract disease clinical research.

### Set of parts in the two-way photodynamic therapy colpostereoscope for diagnosis and treatment of female genital tract diseases.

The new colpostereoscope comprises several parts structuring its systems. These are: mechanical, optical, and electronic parts, which structure 3 assemblies.

### I. - Mechanical Assembly

This set is the frame or skeleton, comprising and supporting the optical and mechanical parts of the equipment:
**Base and support:** Comprised by a balanced steel disc providing stability to the equipment, four or six independent rotary wheels with a brake mechanism for equipment positioning, a vertical stainless steel tube and a horizontal support with a rotating joint for arm and head assembly and maneuvering; light box and system power source electronics are located in the horizontal support above the base disc.
**Arm:** A balanced mechanism which allows three-axis motion. Such motion provides the exact head location at different working heights and positions, giving the possibility to easily maneuver the equipment at the time of a gynecologic procedure.
**Head:** The central and most important part of the equipment. In other words, **this is truly the new colpostereoscope,** housing the following components: focusing system with fine motion mechanism and optical system comprising two filter types, head variable linearity excitation filter and variable linearity suppressor filters. This is the colpostereoscope core where light energy transmission and modulation is carried out which allows cervix image monitoring with different amplifications directly through Galileo drum or through the two high-resolution digital television cameras located at the sides of said head transmitting the image to a monitor in the novel three-dimensional DLP HDTV system.

### II. - Electronic Assembly:

This apparatus works with 240-220 volt current lines (European), 110-120v (American) and 90-100V (Japanese).

A power source to supply metal halide light bulbs, a power source for Xenon light bulbs and the respective ballast for lighting thereof.

A pair of metal halide bulbs is placed in the head, having detectors for inner temperature monitoring and regulation, connected to printed circuits controlling the temperature and regulating the fan blade revolutions, and beeping an alarm at the time that the apparatus has been inadvertently remained in operation in spite of having the cooling system working at maximum.

Control and regulation automatic systems for inner temperature produced by the light bulbs and components are also designed in printed circuits, with their respective cooling fans.

Basic electronic components in optical fiber light box are basically: a high voltage transformer, a ballast, a 6/12-volt, 5/10-amp high amperage low voltage transformer, two Xenon light bulbs (one for use and one spare), two metal halide (halogen) light bulbs (one for use and one spare), a regulated mini-motor for variable linearity filter displacement, potentiometers, integrated circuits and voltage regulators.

### III. - Optical assembly.

### 1. - Lighting Step

The set in the head comprises two metal halide (halogen) light bulbs (one for use and one spare) located in a vertical direction parallel to the collecting lens, with a light bulb positioning mechanism which allows distance adjustment in two axes until the highest light intensity point is obtained, meeting the features of the so-called Kohler illumination microscopy.

The light emitted from the light bulb filament passes through the collecting lens, concentrating the light beam, which is reflected at 90 degrees by a mirror arranged for this purpose at 45 degrees in the light path axis.

It is important to emphasize that this invention consists of episcopic illumination, based on the illumination standards applicable to optics and microscopy, including Kohler illumination which allows the use of light energy with less expense and less heat emission.
1. - Light is emitted by an infinity-focused light source, passing through a positive or converging lens, deflecting the light beams and concentrating them in a focal plane such that a smaller diameter inverted image is provided.
2. - When using two converging lenses, and the second "front lens" lens focuses the image projected by the first "collecting" lens, the light source filament image passes without being seen in the focus focal plane, in this case the cervix; in other words, the focus image is a coiled filament and this image is unnoticed. The filament is not seen, but its energy can be used optimally as its beams in the second focal plane cross in parallel opposite to the projected image of the first converging lens. This is the Kohler principle.

The first lens image is projected onto the focal plane surface in this case, the cervix. Smooth and colorless as the glass, this is seen as a uniform light field. In the light of above, this illumination system was selected in our colpostereoscope. Our next step is:

### 2. - Excitation Step:

A variable linear filter has been chosen which may be adjusted to the selected working light frequency for exciting the desired compound, called a head excitation filter, for just selecting a single wavelength, as in the case of our colpostereoscope. Another excitation filter, called the optical fiber box excitation filter has been similarly assembled in the optical fiber light box.

The following describes the fluorochrome, fluorescent protein, photodynamic diagnosis marker or fluorescent dye excitation in our system.

Firstly, that is applied at the cervix by a suitable technique, and absorbed by the infected tissue cells or in the lesions caused by human papillomavirus. The selected light beam is introduced on the cell tissue compound, thereby the light energy is absorbed in the tissues, and then emits light at a different light wavelength than that irradiated. The physical phenomenon of fluorescence thus arises: what is been looking for in the tissue fluoresces. The light energy irradiated by the excitation filter is absorbed by the compound atoms, exciting their electrons, changing spins, causing photon emission. The photon is produced when an electron changes its spin, photons with a wavelength other than that originally irradiated in cells and tissues (Stokes-Adams principle).

The above described reactions in photodynamic diagnosis application are also present in this step.

### 3. - Suppression Step.

The light and image emitted by the irradiated compound is collected by the front lens and in parallel (one beam for each eye) is transmitted to the variable linearity suppression filters where the light frequency emitted from the compound is selected, modulating and matching with diaphragms of contrast and depth of field located inside the head optical system. Parallel image then passes through a assembled lens set (first eyepieces) in the Galileo drum to choose the desired amplification, sending the image to the Porro prisms which invert the image to be analyzed by the lenses by direct view or observation, or this image is sent instead to each of the two high-definition television cameras located at each of the two visual axes, the image then passes to the three-dimensional vision video system DLP (digital light processing) HDTV (High definition television) and this image is seen in the monitor.

The three-dimensional stereoscopic systems functions in this colpostereoscope at an angle between the two visual axes arranged between 7 to 12 degrees of opening.

### Description of the figures

Figure 1 shows schematically the optical system used in the colpostereoscope head in a single and easy way.
Figure 2 shows the excitation variable linearity suppressor filter assembly used in the optical fiber light box.

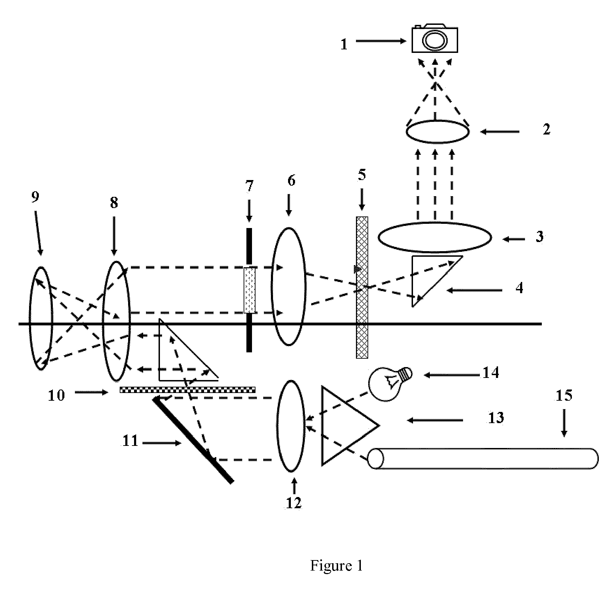

Description of Figure 1: The number 1 indicates the image output where the video cameras or viewer's eyes are positioned, 2 and 3 numbers refer to the eyepiece lens which comprises image upper and receiving lenses, number 4 indicates inverting image prisms in order to view the images in the proper position (these are called Porro prisms). Number 5 indicates where the variable linearity barrier or suppressor filter is placed which is exactly at the light beam intersection which, as stated in the description, is for selecting the desired light spectrum that is emitted by the compound under study; this filter should slide through its axial axis thus choosing the desired frequency. Number 6 shows the interchangeable lens for choosing desired magnifications. Number 7 indicates the place for the contrast and depth of field diaphragm. Numbers 8 and 9 are the shooting lenses, front lens and second shooting lens and so on, these lenses are further used in the colpostereoscope as subject's illuminating light capacitor. Number 10 is the variable linearity excitation filter located where light beams coming from the illuminator converge. In order to choose the required wavelength for exciting the compound which will emit luminescence, the illuminator should only travel its longitudinal axis to select the desired frequency; this is also used in photodynamic therapy application using any drug intended for this purpose. Number 11 is a mirror providing the required focal length for the light emitted by the illuminator which is projected by the condenser collection lens marked with number 12. Number 13 is a prism selector to choose the required illumination pathway which may be emitted by halogen bulbs mounted on head 14 or that originating from the optical fiber 15 box through the distal part thereof.

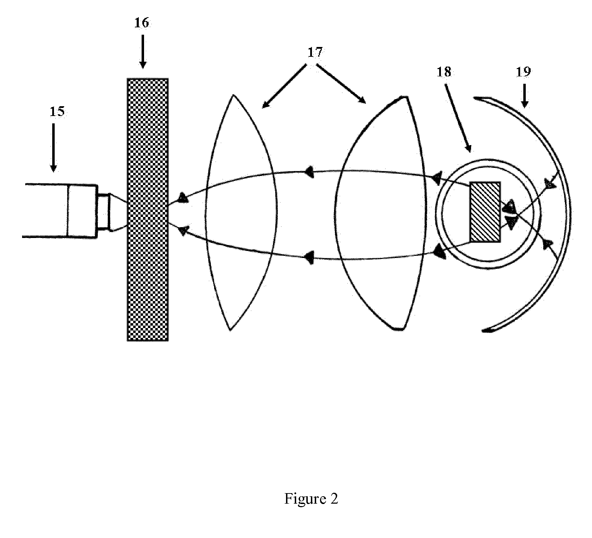

Description of Figure 2: When the optical fiber light box function is chosen, the head mounted excitation filter should be previously left out of the light beam axis in order to manipulate the light frequencies from the box. Optical fiber 15 in its proximal box portion is mounted on a special connector cylinder for this purpose; the variable linearity excitation filter is just located ahead being also slid for photodynamic therapy working excitation frequency choice, this filter is indicated by the number 16 in the drawing, high-power light source collecting converging lenses are indicated by the number 17. Number 18 indicates the xenon or high-power light irradiating lamp. A concave mirror 19 lies backwards to concentrate the light emitted by the lamp from its rear part, condensing and sending it to the front to take full advantage of the emitted energy.

### Examples for the use of a photodynamic diagnosis colpostereoscope (PDD) for treatment of female genital tract diseases and neoplasm lesion early detection.

The examples shown below are non-restrictive and nonlimiting examples in the use of the apparatus when performed in practice, since as previously disclosed, this instrument is designed for multiple current and future applications in the medical field.

The use of the photodynamic diagnosis colpostereoscope may be also useful, prior to introducing the following examples, as common colpostereoscopes with the usual technique of acetic acid test and iodine solution application, and the introduction of green filter for observing the capillary bed in the cervical-vaginal mucosal tissue.

### Example 1. Diagnosis for human papillomavirus lesions or neoplasms using a fluorochrome: Fluorescein Clinical Method.

The fluorescein isothiocyanate (FITC) fluorochrome was used in this case for several reasons:
This is widely used by ophthalmologists in fluoroangiographies (retina), and in local solution to see the corneal lesions. They use it with a lamp called "Actinic Lamp", which has a cobalt filter and the emission spectrum is blue. This lamp used by them has no suppressor filters, we have chosen this fluorochrome since it has no adverse or toxic effects in humans.

### Procedure:

- Place the patient comfortably in gynecologic position. Study duration is 15-20 minutes.
- Introduce a plastic speculum to prevent unwanted reflections.
- Locate the cervix.
- Take vaginal pH (acidity or alkalinity): HPV infection often coexists with other bacterial or parasitic diseases so that the pH is alkaline.
- Take samples from endocervix with cervical brush.
- Take samples of ectocervix with Ayre spatula.
- Prepare evenly slide samples for laboratory, just to confirm our diagnosis, when required.
- Apply 5% acetic acid to the cervix for a minute. This acidic solution has two functions, firstly a cell bleach in cervical intraepithelial neoplasm and secondly, a coupler for fluorochrome (FITC) application with cells.
- Perform a routine colposcopic technique.
- Insert the green filter to see vascular changes.
- Make three-dimensional video-photographic records for comparison.
- Apply evenly FITC (fluorescein isothiocyanate) to the cervix for a minute.
- Select the frequency in the head variable linearity excitation filter in a range of 488 nanometers (blue).
- Select the frequency in variable linearity suppressor filters in a range of 520 nm (yellow).
- Make direct observation or make video-photographic records with three-dimensional cameras mounted on the head.
- When a patient is positive to fluorescein test in detecting neoplasms, the image of the lesion produced by the human papilloma virus will be seen in bright green color on a black background, if the patient were negative the black background would be without green image.

### Example 2. Photodynamic diagnosis (PPD) for human papillomavirus lesions using a photosensitizing agent, 5-aminolevulinic acid (ALA).

### Clinical Method.

### Procedure:

- Place the patient comfortably in lithotomy position.
- Introduce a vaginal speculum.
- Locate the cervix.
- Clean the cervix and vaginal walls with normal saline.
- Apply the cream with the photosensitizer.
- Putting the patient at rest for 2 hours.
- Repeat the first three steps again.
- Select the frequency in the head variable linearity excitation filter to a range of 405 nanometers (blue-violet).
- Select the variable linearity suppressor filters at a range of 635 nanometers (red).
- Make direct observation or make video-photographic records with three-dimensional cameras mounted on the head.

When a patient is positive to photodynamic diagnosis (PPD), the lesion image is seen in bright red on a black background, if the patient is negative the black background is simply seen without red image.

Having sufficiently described the photodynamic diagnosis colpostereoscope (PDD) for female genital tract diseases and neoplasm lesion early detection and as our invention is considered novel, we claim for our exclusive property the contents of the following claims:

## Claims

1. **Photodynamic diagnosis colpostereoscope (PDD) for female genital tract diseases and neoplasm lesion early detection, characterized by** a lighting system in the head having a variable linear type excitation filter system, two suppressors or barrier filters being placed on said head, one for each observation optical axis; two diaphragms have been placed on the head, one on each optical axis with a manual opening controller. The three-dimensional observation in this colpostereoscope may be by direct observation through the eyepiece as usually done in these apparatuses, or with a novel way of having two high resolution video cameras placed on said head, each camera being placed in each visual axis. The three-dimensional video vision system is: DLP (digital light processing) HDTV (high definition television) with the use of three-dimensional active lenses. A light source box with focusable optical system and directed to an optical fiber is installed in the colpostereoscope. Episcopic illumination which is based on the lighting standards applied in microscopy and optics and based on Kohler illumination, is also included in this invention.

2. **Photodynamic diagnosis colpostereoscope (PDD) for female genital tract diseases and neoplasm lesion early detection** according to claim 1, **characterized by** a lighting system in the head having a variable linear type excitation filter system capable of selecting any light frequency range to send a light beam to any fluorescent compound placed in the female genital tract, these compounds may be: fluorochromes, photosensitizers, fluorescent proteins of any color for "in vivo" use, or chemical dyes for fluorescence. This filter spans a range from 400 nanometers to 1080 nanometers, the light beam band may be chosen every 20 nanometers.

3. **Photodynamic diagnosis colpostereoscope (PDD) for female genital tract diseases and neoplasm lesion early detection** according to claim 1, **characterized by** having two variable linearity barrier or suppressor filters in the head, one for each observation optical axis to analyze the frequency emitted by the fluorescent compounds and to eliminate the wavelengths produced by the excitation light. These filters are also for accurate adjustment of emission rays emanating from the compound to react in the dyed female genital tract. These filters span a light spectrum from 400 to 700 nanometers, the visible light spectrum, and the frequencies can be staggered every 20 nanometers.

4. **Photodynamic diagnosis colpostereoscope (PDD) for female genital tract diseases and neoplasm lesion early detection** according to claim 1, **characterized by** having two diaphragms in the head, one on each optical axis with a manual opening controller capable of contrasting the image of what is being observed and also watching the depth of field.

5. **Photodynamic diagnosis colpostereoscope (PDD) for female genital tract diseases and neoplasm lesion early detection** according to claim 1, **characterized by** having two high resolution video cameras placed in the head, each camera is placed on each visual axis. The three-dimensional vision video system is: DLP (digital light processing) HDTV (high definition television) with the use of three-dimensional active lenses.

6. **Photodynamic diagnosis colpostereoscope (PDD) for female genital tract diseases and neoplasm lesion early detection** according to claim 1, **characterized by** a light source box with focusable optical system and directed to a medical grade optical fiber or to the novel low cost and high quality optical fiber made of ethylene and propylene fluorinate (FEP) resin. A variable linearity filter is included within the optical system as the head, in order to select any light frequency range to send a light beam through the optical fiber to any compound or drug located in the female genital tract, using exploration by photodynamic diagnosis, this filter spanning a range from 400 nanometers to 700 nanometers, the light beam band which may be chosen every 20 nanometers thus providing a virtually universal equipment application to any drug or compound targeted for photodynamic diagnosis genital tract gynecologic exploration.

7. **Photodynamic diagnosis colpostereoscope (PDD) for female genital tract diseases and neoplasm lesion early detection** according to claim 1, **characterized by** including episcopic illumination, which is based on the lighting standards applicable to microscopy and optics based on Kohler illumination, which allows the use of light energy with less expense and less heat emission.
